# EUROPÄISCHE PATENTSCHRIFT

(11) **EP 3 737 341 B1**
(45) Veröffentlichungstag und Bekanntmachung des Hinweises auf die Patenterteilung: **03.07.2024**
(21) Anmeldenummer: 19700259.5
(22) Anmeldetag: 08.01.2019
(51) Int. Cl.: A61F 2/64, A61F 2/60

(54) **ORTHOPÄDIETECHNISCHE VORRICHTUNG**
ORTHOPAEDIC DEVICE
DISPOSITIF ORTHOPÉDIQUE

(30) Priorität: 08.01.2018 DE 102018100252
(43) Veröffentlichungstag der Anmeldung: 18.11.2020
(73) Patentinhaber: Otto Bock Healthcare Products GmbH, 1110 Wien (AT)
(72) Erfinder: RUMPLER, Julian, 2801 Katzelsdorf (AT); BODENSTEIN, Kay, 1140 Wien (AT); MEJIA NINO, Juan Pablo, 1160 Wien (AT)
(74) Vertreter: Gramm, Lins & Partner Patent- und Rechtsanwälte PartGmbB
(86) Internationale Anmeldenummer: PCT/EP2019/050296
(87) Internationale Veröffentlichungsnummer: WO 2019/135005

(56) Entgegenhaltungen:
- WO-A1-2017/050552
- DE-A1-102009 004 950
- DE-A1-102016 202 287
- US-A- 2 657 393
- US-A1- 2008 228 287

## Beschreibung

Die Erfindung betrifft eine orthopädietechnische Vorrichtung mit einer hydraulischen Dämpfeinrichtung, mit einem Ventil mit einem Ventilsitz und mit einem in Richtung auf den Ventilsitz über eine vorgespannte Ventilfeder mit einer Schließkraft beaufschlagten Ventilkörper und einer strömungstechnischen Verbindung zwischen der hydraulischen Dämpfeinrichtung und dem Ventilsitz.

Orthopädietechnische Vorrichtungen, insbesondere Prothesen oder Orthesen, können mit einer hydraulischen Dämpfeinrichtung ausgestattet sein, die einen Kolben und einen Zylinder aufweist, wobei der Kolben den Zylinder in der Regel in eine Extensionskammer und eine Flexionskammer unterteilt, wenn die hydraulischen Dämpfeinrichtung an einem Gelenk angeordnet ist. Je nach Extensions- oder Flexionsbewegung wird Hydraulikfluid von der einen Kammer in die andere Kammer bewegt. Bei einer anderen Anordnung werden die beiden Kammern als Kompressionskammer und Extensionskammer bezeichnet. Bei hydraulisch gedämpften Gelenkeinrichtungen sind häufig verstellbare Ventile als Strömungsventil ausgebildet, über die entweder veränderlich oder statisch die Widerstände in dem jeweiligen Strömungskanal eingestellt werden können. Die Strömungsventile können manuell einstellbar oder motorisch einstellbar sein. Darüber hinaus sind in hydraulischen Dämpfeinrichtungen Rückschlagventile angeordnet, die strömungsrichtungsabhängig den Fluidstrom sperren. Um den Ventilkörper in der gewünschten Position zu halten, sind diese Ventile in der Regel mit einer Feder belastet, die den Ventilkörper in den Ventilsitz vorspannt. Damit das Hydraulikfluid durch das Rückschlagventil hindurchströmen kann, muss ein Druck in Richtung der Federkraft entgegengerichtet anliegen, damit der Ventilkörper von dem Ventilsitz abgehoben wird.

In hydraulischen Dämpfeinrichtungen sind teilweise sogenannte Sicherheitsventile eingebaut, die bei Überschreiten eines maximal zulässigen Druckes öffnen, um die orthopädietechnische Vorrichtung, insbesondere die hydraulische Dämpfeinrichtung, vor mechanischen Schäden oder auch den Patienten vor Verletzungen zu schützen. Das Sicherheitsventil ist eine Überlastsicherung, das bei Erreichen eines vorgegebenen Druckes öffnet. Insbesondere bei orthopädietechnischen Vorrichtungen mit einer hydraulischen Dämpfeinrichtung ist der Bauraum begrenzt. Dennoch müssen über kleine Ventilfedern, die direkt oder indirekt auf den Ventilkörper eine Vorspannkraft ausüben, hohe Kräfte aufgebracht werden. Insbesondere bei einer Ausgestaltung der Ventilfeder als eine mechanische Schraubenfeder oder Wendelfeder besteht das Problem, dass eine große Fertigungsstreuung vorliegt, so dass eine gewünschte Ventilöffnungskraft nur über das Ausprobieren einer großen Anzahl von Ventilfedern erfolgen kann.

Die US 2 657 393 A betrifft eine fluidgedämpfte Protheseneinrichtung mit einem einstellbaren Widerstandselement in Gestalt eines federbelasteten Ventils in einem Strömungskanal.

Die US 2008/228287 A1 betrifft ein Prothesenkniegelenk mit einem Hydraulikdämpfer mit einem Kolben, der in einem Zylinder verlagerbar gelagert ist. In einem Strömungskanal ist ein einstellbar federvorgespanntes Ventil angeordnet.

Die DE 10 2009 004 950 A1 betrifft ein künstliches Kniegelenk mit drehmomentfreier Standphasensteuerung und einem Hydraulikdämpfer mit einem absperrbaren Magnetventil und einer einstellbaren Ansprechschwellenfeder. In Abhängigkeit von der Bodenreaktionskraft im Verhältnis zu der eingestellten Vorspannkraft der Ansprechfeder wird ein elektromechanischer Wechsler in unterschiedliche Schaltstellungen bewegt.

Die DE 10 2016 202 287 A1 betrifft einen Hydraulikdämpfer einer prothetischen Gelenkeinrichtung mit einem Ventil, das ein Pinselelement aufweist, das bei einer Verrückung aus der senkrechten Position Flüssigkeit in Bezug auf die entsprechende Pendelventilsitzfläche bei nicht senkrechter Ausrichtung durchlässt. Das Pendelelement kann federgespannt sein, es lässt sich so abändern, dass damit unterschiedliche Federwirkungen bereitgestellt werden können. Das federgespannte Element kann mit dem Boden auf einem verstellbaren Sitzteil angebracht sein.

Die WO 2017/050552 A1 betrifft eine orthopädietechnische Gelenkeinrichtung mit einem Oberteil und einem gelenkig daran befestigten Unterteil und einer zwischen dem Oberteil und dem Unterteil angeordneten Halteeinrichtung. Die Halteeinrichtung ist flexionsmomentgesteuert ausgebildet und sperrt eine Flexion so lange, bis ein vorbestimmtes Flexionsmoment die Flexion freigibt. Der orthopädietechnischen Gelenkeinrichtung kann ein Hydraulikdämpfer zugeordnet sein, bei dem in einem Überströmkanal ein Rückschlagventil angeordnet ist, das federbelastet ist. Die Federvorspannung ist über eine Einstelleinrichtung einstellbar.

Aufgabe der vorliegenden Erfindung ist es daher, eine orthopädietechnische Vorrichtung bereitzustellen, bei der immer die gewünschte Ventilöffnungskraft vorhanden ist.

Erfindungsgemäß wird diese Aufgabe durch eine Vorrichtung mit den Merkmalen des Hauptanspruches gelöst. Vorteilhafte Ausgestaltungen und Weiterbildungen der Erfindung sind in den Unteransprüchen, in der Beschreibung und in den Figuren offenbart.

Die erfindungsgemäße orthopädietechnische Vorrichtung mit einer hydraulischen Dämpfeinrichtung, mit einem Ventil mit einem Ventilsitz und einem in Richtung auf den Ventilsitz über eine vorgespannte Ventilfeder mit einer Schließkraft beaufschlagten Ventilkörper und einer strömungstechnischen Verbindung zwischen der hydraulischen Dämpfeinrichtung und dem Ventilsitz, sieht vor, dass das Ventil eine Verstelleinrichtung zum Verstellen der Vorspannung die der Ventilfeder aufweist. Während bei den Überdruckventilen aus dem Stand der Technik eine fixe Einstellung und eine große Variation der jeweiligen Öffnungskräfte aufgrund unterschiedlicher Federsteifigkeiten vorliegt, ist es mit der erfindungsgemäßen Vorrichtung möglich, auch bei Vorliegen unterschiedlicher Federsteifigkeiten der jeweiligen Ventilfedern eine stets gleiche Schließkraft des Ventilkörpers bereitzustellen, so dass unabhängig von den Fertigungstoleranzen der Ventilfeder eine gleichbleibende Qualität der orthopädietechnischen Vorrichtung bereitgestellt werden kann. Darüber hinaus ist es möglich, eine Individualisierung der orthopädietechnischen Vorrichtung an den jeweiligen Nutzer oder Patienten vorzunehmen. Beispielsweise bei orthopädietechnischen Vorrichtungen wie Orthesen oder Prothesen der unteren Extremitäten kann die notwendige Schließkraft mit dem Körpergewicht oder mit dem Belastungsgewicht auf das jeweilige Gelenk variieren. Ein leichter Patient kann eine geringere Federkraft zum Öffnen des Ventils benötigen, so dass die Gelenkeinrichtung nachgibt, wenn eine spezifische Belastung vorliegt, während bei einem schwereren Patienten oder bei einer größeren Belastung, beispielsweise wenn der Patient Lasten trägt, eine höhere Schließkraft eingestellt werden kann. Die Einstellung der Schließkraft über eine Veränderung der Ventilfedervorspannung ermöglicht zudem eine Anpassung des Ventils an unterschiedliche orthopädietechnische Vorrichtungen mit unterschiedlichen Einsatzzwecken und beispielsweise unterschiedlichen Modellgrößen.

Eine Weiterbildung der Erfindung sieht vor, dass die Verstelleinrichtung ein Gewindetrieb aufweist, über den die Ventilfeder vorspannbar ist. Die Ventilfeder selbst kann als mechanische Feder wie Wendelfeder oder Schraubenfeder ausgebildet sein und über insbesondere eine Druckkraft oder auch eine Zugkraft den Ventilkörper in den Ventilsitz drücken oder ziehen. Ebenfalls ist es möglich, alternative Ventilfedern vorzusehen, beispielsweise ein Elastomerelement oder ein vorgespanntes, kompressibles Volumen, beispielsweise eine mit Gas gefüllte Blase. Der Ventilkörper kann in direktem Kontakt mit der Ventilfeder stehen, alternativ kann die Schließkraft des Ventilkörpers über ein Trägerelement oder ein bevorzugt inkompressibles Fluid auf den Ventilkörper aufgebracht werden, um den Ventilkörper in den Ventilsitz zu pressen.

Die Verstelleinrichtung kann einen mit der Ventilfeder gekoppelten Ventilfederhalter aufweisen, der in einem Gehäuse gelagert und an dem ein erstes Gewinde ausgebildet ist, das mit einem zweiten Gewinde, das in dem Gehäuse angeordnet ist, in Eingriff steht. Die Verstelleinrichtung kann zusammen mit zumindest der Ventilfeder und dem Ventilkörper zusammen mit dem Ventilfederhalter in einem separaten Gehäuse eingesetzt und als Modul ausgebildet sein. Alternativ kann das Gehäuse Teil des Gehäuses der orthopädietechnischen Vorrichtung sein, beispielsweise ein Strömungskanal, in dem ein Innengewinde angeordnet oder ausgebildet ist, das mit einem Gewinde eines Ventilfederhalters, in der die Ventilfeder vorspannt oder entspannt, in Eingriff gebracht werden kann. Der Ventilfederhalter kann beispielsweise als Zapfen, Hülse oder eine ähnliche Aufnahmeeinrichtung ausgebildet sein, an der oder in der die Ventilfeder gelagert ist und über den die Ventilfeder gespannt oder entspannt werden kann. Je nachdem, in welche Richtung die Verstelleinrichtung bewegt wird, wird über den Gewindetrieb die Ventilfeder vorgespannt oder entspannt.

Der Ventilkörper kann mit einer Vorspannkraft in Richtung auf den Ventilsitz vorgespannt sein, die eine nahezu beliebige Höhe aufweisen kann. Je nachdem, welche Schließkraft bereitgestellt werden muss, um das hydraulische System zu schützen oder die hydraulische Dämpfeinrichtung an den jeweiligen Patienten oder die jeweilige Einsatzbedingung anzupassen, kann die Vorspannkraft eingestellt und durch die Verstelleinrichtung angepasst werden. Bevorzugt ist die Vorspannkraft stufenlos einstellbar, um Abweichungen oder Streuungen in der Fertigung der Federn ausgleichen und genaue Anpassungen an den Anwender und/oder den Einsatzzweck vornehmen zu können. Durch die stufenlose Einstellbarkeit können Streuungen deutlich reduziert und die Vorspannkraft mit sehr geringen Abweichungen an den Sollwert eingestellt werden. Die Abweichungen von dem Sollwert betragen bevorzugt weniger als 10%, insbesondere bevorzugt weniger als 5%, beispielsweise bei einem Sollwert von 9kN kann mit einer Genauigkeit von ±0,4kN die Vorspannkraft eingestellt werden.

Die Verstelleinrichtung kann Einstellmarkierungen aufweisen, so dass der Nutzer oder der Orthopädiemechaniker die jeweils gewünschte Schließkraft anhand der Einstellmarkierungen einstellen kann. Nachdem Einbau der Feder kann eine Prüfkraft auf den Ventilkörper aufgebracht werden. Öffnet das Ventil vor Erreichen der eingestellten Prüfkraft, wird die Vorspannung erhöht, öffnet das Ventil noch nicht, wird die Federvorspannung verringert und damit auch die Schließkraft verringert. Über eine Abstufung der jeweils aufgebrachten Prüfkraft und das Anbringen der jeweiligen Einstellmarkierung bei Erreichen der gewünschten Prüfkraft kann für jede Feder individuell die jeweilige Einstellmarkierung oder die jeweiligen Einstellmarkierungen aufgebracht werden.

Die Verstelleinrichtung weist bevorzugt ein Formschlusselement auf, über das die Verstelleinrichtung betätigbar ist. Das Formschlusselement kann beispielsweise als Schlitz, Kreuzschlitz, Polygonalprofil, Torxeinsatz, Innensechskant oder als korrespondierender Vorsprung eine Kraftübertragung von einem Werkzeug oder einem motorischen Antrieb auf die Verstelleinrichtung ermöglichen.

In der Erfindung ist es vorgesehen, dass die Verstelleinrichtung einen motorischen Antrieb und ein mit dem motorischen Antrieb gekoppeltes Steuergerät zum Steuern des motorischen Antriebs aufweist. Der Antrieb ist insbesondere als elektromotorischer Antrieb ausgebildet. Alternative motorische Antriebseinrichtungen zum Verstellen der Vorspannung der Ventilfeder sind möglich und vorgesehen. Über den motorischen Antrieb können ohne einen direkten Zugang zu der Verstelleinrichtung durch Aktivierung oder Deaktivierung des Antriebes die jeweils gewünschte Verstellung in der jeweils gewünschten Richtung vorgenommen werden.

Bevorzugt weist die orthopädietechnische Vorrichtung ein mit dem Steuergerät gekoppeltes Bedienelement und/oder eine Kommunikationsstelle zum Ansteuern des motorischen Antriebs auf, so dass über ein Fernbedienung oder über ein Bedienelement an der Orthese, Prothese oder dem Rollstuhl oder einer anderen orthopädietechnischen Einrichtung bequem die Verstellung der jeweiligen Federvorspannung erreicht werden kann.

Die Erfindung sieht vor, dass die orthopädietechnische Vorrichtung zumindest einen Sensor aufweist, der mit dem Steuergerät gekoppelt ist. Über den Sensor ist es möglich, das Steuergerät mit Daten des Nutzers oder auch mit Nutzungsdaten der orthopädietechnischen Vorrichtung zu versorgen, um die Federvorspannung und damit den Schließdruck oder den Druck, ab dem der Ventilkörper das Ventil öffnet, einstellen zu können. In einer direkten Einstellung durch den Nutzer der Dämpfeinrichtung oder einen Orthopädietechniker über eine manuelle Verstellung oder über eine motorische Verstellung mittels einer Schnittstelle oder ein Bedienelement ist es möglich, eine Einstellung aktiv über den Sensor für jeden Nutzungszustand oder auch für eine spezielle Aktivität oder auch für Veränderungen an dem Nutzer automatisch durchführen zu lassen. Für spezielle Aktivitäten wie Sport, das Tragen von Gewichten oder für Sonderfunktionen wie Bergabgehen oder das Herabsteigen von Treppen kann es sinnvoll sein, besondere Schließdrücke oder Grenzwerte einzustellen, damit das Hydrauliksystem bei Belastungsspitzen keine Schäden davonträgt oder um zu verhindern, dass zu große Kräfte über die prothetische oder orthetische Vorrichtung auf den Patienten übertragen werden. Das jeweilige Gelenk oder die jeweilige orthopädietechnische Vorrichtung gibt dann bei Erreichen des Grenzdruckes nach, bei Gelenken der unteren Extremität überwiegend bei der Flexion eines Knies oder eines Knöchelgelenkes, bei einem Knöchelgelenk zudem auch bei einer Extensionsbewegung.

Über eine aktive Steuerung mit einem Steuergerät, das die Vorspannung in Abhängigkeit zumindest eines Sensorwertes verstellt, kann eine verbesserte Individualisierung und Anpassung an den Patienten oder die jeweilige Aktivität durchgeführt werden.

In einer Weiterbildung der Erfindung ist es vorgesehen, dass das Steuergerät dazu eingerichtet ist, das Körpergewicht und/oder ein Wert eines Parameters einer Bewegung mittels zumindest eines Sensors zu ermitteln, aus dem ermittelten Wert oder den ermittelten Werten zumindest einen Steuerparameter für den Antrieb zu errechnen und automatisch den Antrieb zum Verstellen der Vorspannung der Ventilfeder zu aktivieren. Insbesondere die Gewichtsbelastung, beispielsweise über einen Axialkraftsensor, kann gemessen werden, ebenso Beschleunigungen, Momente oder Raumlagen, auf deren Grundlage automatisch das Ventil verstellt wird.

Nachfolgend werden Ausführungsbeispiele der Erfindung anhand der beigefügten Figuren näher erläutert. Es zeigen:
- Figur 1-: eine schematische Schnittdarstellung eines Ventils;
- Figur 2 -: eine Variante der Figur 1 mit einer motorischen Verstelleinrichtung;
- Figur 3 -: eine Schnittdarstellung durch einen Hydraulikdämpfer mit dem Ventil; sowie
- Figur 4 -: eine schematische Darstellung eines Anwendungsbeispiels.

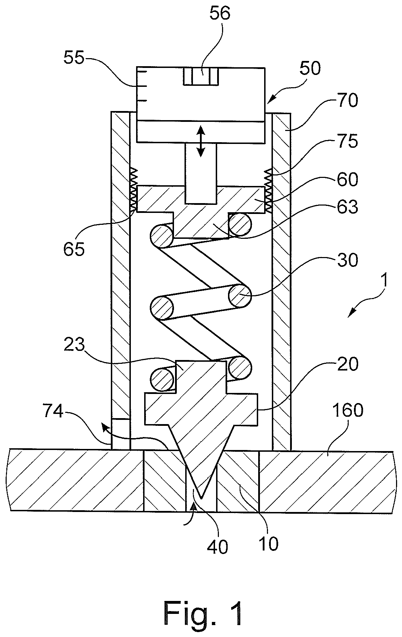

In der Figur 1 ist in einer schematischen Schnittdarstellung ein Ventil 1 in einer Schnittdarstellung gezeigt, mit einem Ventilsitz 10, der in einem Teil einer hydraulischen Dämpfereinrichtung oder einem anderen hydraulischen System angeordnet ist. Der Ventilsitz 10 ist in dem dargestellten Ausführungsbeispiel in einem Hydraulikkolben 160 angeordnet, der später näher erläutert wird. In dem Ventilsitz 10 ist eine Durchgangsbohrung 40 oder ein Durchlass ausgebildet, der eine Hochdruckseite mit einer Niederdruckseite verbindet. Der Durchlass 40 ist durch einen Ventilkörper 20 verschlossen. Der Ventilkörper 20 weist in dem dargestellten Ausführungsbeispiel eine Kegelspitze auf, die in den Durchlass 40 hineinragt und diesen verschließt. In dem Ventilsitz 10 können Anlageschrägen ausgebildet sein, um eine Vergrößerung der Auflagefläche des Ventilkörpers 20 an oder in dem Ventilsitz 10 zu erreichen. Der Ventilkörper 20 ist über eine Feder 30, die in dem dargestellten Ausführungsbeispiel als eine Schraubenfeder oder Wendelfeder ausgebildet ist, gegen den Ventilsitz 10 mit einer Vorspannkraft beaufschlagt. Der Ventilkörper 20 weist einen Vorsprung oder bolzenartigen Absatz 23 auf, um den herum die Schraubenfeder 30 angeordnet ist. Der Absatz 23 dient als Führung für die Schraubenfeder 30.

Die Schraubenfeder 30 zusammen mit dem Ventilkörper 20 sind in einer Hülse 70 geführt, die an dem Kolben 160 angeordnet ist. Die Hülse 70 kann Teil des Kolbens 160 sein oder daran befestigt sein. Die Hülse 70 umgibt sowohl den Ventilsitz 10 als auch den Ventilkörper 20 und die Feder 30 und weist einen Auslass 74 auf, durch den Hydraulikfluid oder gegebenenfalls Pneumatikfluid von der Hochdruckseite durch den Durchlass 40 bei einem geöffneten Ventil 1 austreten kann. Innerhalb der Hülse 70 ist ein Ventilfederhalter 60 angeordnet, der ebenfalls einen bolzenartigen Absatz 63 aufweist, um den herum die Schraubenfeder 30 gelagert ist. Der Ventilfederhalter 60 weist an seiner Außenseite ein Gewinde 65 auf, das in ein Innengewinde 75 an der Innenwand der Hülse 70 eingreift. Über die Gewinde 65, 75 ist es möglich, durch Verdrehen des Ventilhalters 60 in die eine oder andere Richtung die Feder 30 zusammenzudrücken oder zu entspannen. Wird der Ventilfederhalter 60 in Richtung auf den Ventilkörper 20 verlagert, wird die Feder 30 zusammengepresst und die Schließkraft, mit der der Ventilkörper 20 gegen den Ventilsitz 10 gedrückt wird und den Durchlass 40 verschließt, erhöht. Wird der Ventilfederhalter 60 von dem Ventilkörper 20 weg verlagert, entspannt sich die Feder 30 und der Schließdruck wird verringert. Dies bedeutet, dass nur ein geringerer Druck auf der Hochdruckseite benötigt wird, um den Ventilkörper 20 von dem Ventilsitz 10 anzuheben und den Durchlass 40 zu öffnen, so dass Hydraulikfluid und/oder Pneumatikfluid von der Hochdruckseite hindurchtreten kann und durch den Auslass 74 entweichen kann. Der Auslass 74 kann mit einem Ausgleichsbehälter, der Hochdruckseite oder mit einer Verbindungsleitung zu dem übrigen hydraulischen System gekoppelt sein.

Zentral innerhalb des Ventilfederhalters 60 ist eine unrunde Ausnehmung angeordnet, in die ein korrespondierend geformter Zapfen einer Verstelleinrichtung 50 eingreift. An der dem Ventilfederhalter 60 abgewandten Seite der Verstelleinrichtung 50 ist ein Formschlusselement 56 in Gestalt einer Innensechskantausnehmung ausgebildet, in die ein korrespondierend geformtes Werkzeug einführbar ist. Durch Verdrehen der Verstelleinrichtung 50 in die eine oder andere Richtung wird über die Formschlusskopplung mit dem Ventilfederhalter 60 eine Entspannung oder Erhöhung der Vorspannung der Ventilfeder 30 erreicht. An der Außenseite der Verstelleinrichtung 50 sind Markierungen 55 angeordnet, über die die einstellende Person erkennen kann, wie weit eine Vorspannung erfolgt ist. Über die Markierungen können Abstufungen der Vorspannung erkannt werden, so dass die Vorspannkraft nicht nur qualitativ, sondern auch quantitativ kontrollierbar einstellbar ist.

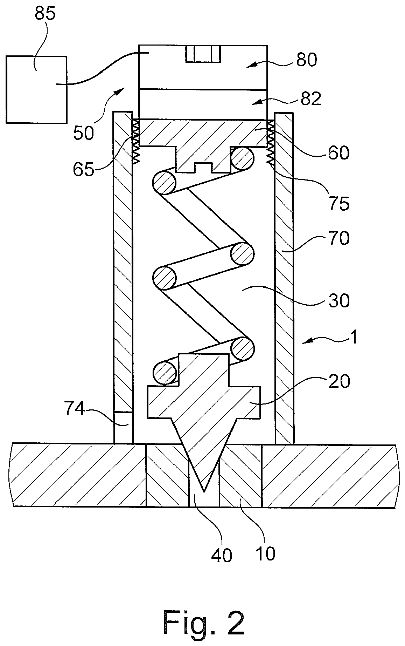

Figur 2 zeigt eine Variante der Ventilanordnung gemäß Figur 1, wobei statt einer mechanischen, handbetriebenen Verstelleinrichtung 50 über ein Werkzeug eine motorisch angetriebene Verstelleinrichtung 50 mit dem Ventilfederhalter 60 gekoppelt ist. Der grundsätzliche mechanische Aufbau mit Ventilsitz 10, Ventilkörper 20, der Vorspannfeder 30 und der Vorspannung über die Gewinde 65, 75 ist unverändert geblieben. An dem dem Ventilkörper 20 entfernten Ende des Ventilfederhalters 60 ist ein Antrieb 80, insbesondere ein elektromotorischer Antrieb 80 angeordnet, der über ein Getriebe 82 mit dem Ventilfederhalter 60 gekoppelt ist. Der motorische Antrieb 80 ist mit einer Steuerungseinrichtung 85 gekoppelt, die wiederum mit zumindest einem Sensor, einer Bedieneinrichtung oder einer Kommunikationseinrichtung gekoppelt sein kann, um über entsprechende Steuersignale den Motor 80 in die eine oder andere Richtung anzutreiben. Durch Verdrehen des Ventilfederhalters 60 innerhalb der Hülse 80 wird durch den Gewindetrieb eine axiale Verlagerung des Ventilfederhalters 60 in Richtung auf den Ventilkörper 20 oder davon weg und damit einhergehend eine Erhöhung der Vorspannung oder eine Entspannung der Feder 30 bewirkt.

Das Getriebe 82 kann insbesondere als Spindelantrieb angeordnet oder ausgebildet sein, so dass der Motor 80 selbst stationär bleibt und lediglich über eine Abtriebswelle das Getriebe 82 antreibt, um über den Ventilfederhalter 60 eine Kompression oder Entspannung der Feder 30 zu bewirken. Andere Verstelleinrichtungen 50 können ebenfalls vorgesehen sein, beispielsweise reine Linearverstelleinrichtungen, die ohne eine Gewindeübersetzung auskommen. Das Steuergerät 85 kann per Kabel oder auch kabellos mit dem Antrieb 80 gekoppelt sein.

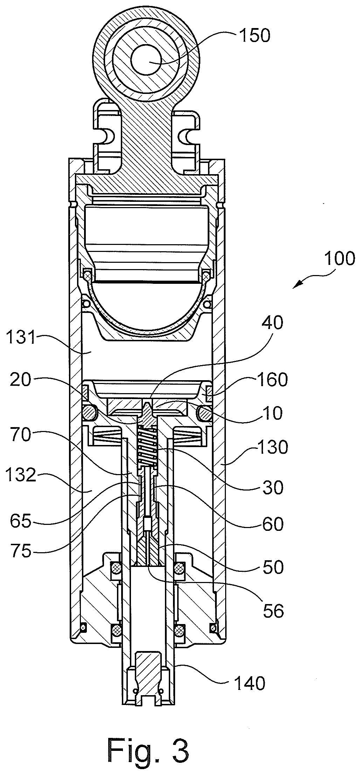

Figur 3 zeigt eine Schnittansicht eines hydraulischen Systems in Gestalt einer hydraulischen Dämpfereinrichtung 100, der das Steuergerät 85 aus der Figur 2 zugeordnet sein kann. Der Hydraulikaktuator 100 weist ein Gehäuse 130 auf, an dem eine Lageraufnahme 150 zur Festlegung des Gehäuses beispielsweise an einer Orthese oder Prothese befestigt ist. Innerhalb des Gehäuses 130 ist ein Zylinder ausgebildet, in dem an einer Kolbenstange 140 ein Hydraulikkolben 160 längsbeweglich angeordnet ist. Der Hydraulikkolben 160 trennt eine Flexionskammer 131 von einer Extensionskammer 132. An dem dem Kolben 160 abgewandten Ende der Kolbenstange 140 kann eine weitere Lagerungseinrichtung vorgesehen sein, analog der Lageraufnahme 150 an dem Gehäuse 130, um den Hydraulikaktuator 100 beispielsweise an einem Unterteil oder einem Oberteil einer Orthese oder Prothese befestigen zu können.

Innerhalb des längsverschieblichen Kolbens 160 ist ein Ventilsitz 10 ausgebildet, in dem der Ventilkörper 20 eingesetzt ist. Auf der Kolbenstangenseite des Kolbens 160 ist in einer Ausnehmung, insbesondere einer zylindrischen Bohrung, der im Querschnitt ebenfalls kreisförmige Ventilkörper 20 eingesetzt und über die Ventilfeder 30 in Richtung auf den Ventilsitz 10 belastet. Innerhalb der Hülse 70, die in dem Ausführungsbeispiel Teil des Kolbens 160 ist und die Bohrung umgibt, ist ebenfalls der Ventilfederhalter 60 angeordnet, der an seiner Außenseite das Außengewinde 65 aufweist, das korrespondierend zu einem Innengewinde 75 in der Hülse 70 ausgebildet ist. Über eine Verstelleinrichtung 50, die an ihrem rückwärtigen Ende ein Formschlusselement 56 in Gestalt einer unrunden oder eckigen Ausnehmung aufweist, kann durch die Kolbenstange 140 eine manuelle Verstellung der Federvorspannung erreicht werden, indem die Verstelleinrichtung 50 und damit auch der über den Gewindetrieb axial verstellbare Ventilfederhalter 60 verlagert wird. Je nach Drehrichtung wird die Feder 30 entspannt oder gespannt. Tritt im Verlauf der Betätigung des Hydraulikdämpfers 100 bei einer Flexionsbewegung eine Überlastspitze in der Flexionskammer 131 auf, öffnet das Ventil 1, indem der Ventilkörper 30 aus dem Ventilsitz 10 entgegen der Federkraft durch die Feder 30 hinausgedrückt wird, so dass der Durchlass 40 geöffnet wird und Hydraulikfluid von der Flexionskammer 131 durch nicht dargestellte Kanäle in die Extensionskammer 132 überströmen kann. Grundsätzlich ist es auch möglich, die Flexionskammer 131 und die Extensionskammer 132 zu vertauschen, so dass bei einem hohen Druck bei einer Extensionsbewegung das Ventil 1 gesteuert geöffnet werden kann. Über die Verstellbarkeit der Vorspannfeder 30 ist es möglich, einerseits den Öffnungsdruck des Ventilkörpers 20 präzisier einzustellen und andererseits durch eine Verstellung der Vorspannung während des Benutzens auf unterschiedliche Aktivitäten, unterschiedliche Gangsituationen oder auf Veränderungen in dem Nutzer der orthopädietechnischen Vorrichtung einzugehen und eine entsprechende Anpassung vorzunehmen.

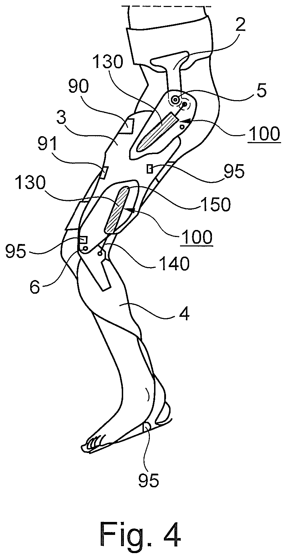

Ein Ausführungsbeispiel der Erfindung ist in der Figur 4 dargestellt, in der eine Orthese für die untere Extremität dargestellt ist. Über einen Hüftgürtel oder einen Hüftanschluss 2 erfolgt eine Festlegung der Orthese an dem Rumpf des Patienten. Über eine Gelenkachse 5 ist eine Oberschenkelschale 3, die über Gurte als Befestigungseinrichtungen an dem Oberschenkel festlegbar ist, an dem Hüftanschluss 2 festgelegt. Distal zu der Oberschenkelschale 3 ist eine Unterschenkelschale 4 mit einer Fußauflage gelenkig über die Schwenkachse 6 an der Oberschenkelschale 3 gelagert. Insgesamt sind zwei Hydraulikaktuatoren 100 vorgesehen, die jeweils zwischen einem Oberteil und einem Unterteil der Orthese angeordnet sind. Zwischen dem Hüftanschluss 2 und der Oberschenkelschale 3 ist der Hydraulikaktuator 100 so festgelegt, dass bei einer Verschwenkung um die Schwenkachse 5 die Kolbenstange 140 in das Gehäuse 130 einfährt bzw. ausfährt. Entsprechend ist ein zweiter Hydraulikaktuator 100 mit dem Gehäuse über die Lageraufnahme 150 an der Oberschenkelschale und über die Kolbenstange 140 und der Befestigungsstelle an der Unterschenkelschale oder dem Unterschenkelteil 4 gelagert. Auch hier führt eine Verschwenkung um die Schwenkachse 6 zu einer Relativbewegung zwischen der Kolbenstange 140 und dem Gehäuse 130 und damit zu einer Bewegung des Kolbens 160 innerhalb des Gehäuses 130. An der Orthese sind Sensoren 95 angeordnet, beispielsweise Drucksensoren, Beschleunigungssensoren, Kraftsensoren oder Winkelsensoren, die mit dem Steuergerät 85 gekoppelt sind. Ebenfalls ist in dem dargestellten Ausführungsbeispiel an der Oberschenkelschale 3 ein Bedienelement 90 z.B. in Gestalt eines Touch Screens angeordnet, über das es möglich ist, das Steuergerät 85 und damit den motorischen Antrieb 80 zum Verstellen der Vorspannung des Ventils 1 zu bedienen. Ergänzend ist eine Kommunikationsschnittstelle 91 an der Orthese, in dem Ausführungsbeispiel an der Oberschenkelschale 3 angeordnet, um beispielsweise über eine zentrale Steuereinrichtung ein Softwareupdate aufzuspielen oder Daten und Energie aufzunehmen und an die orthopädietechnische Vorrichtung mit der Hydraulikeinheit weiterzugeben oder eine Datenauswertung zu betreiben.

## Patentansprüche

1. Orthopädietechnische Vorrichtung mit
einer hydraulischen Dämpfereinrichtung,
einem Ventil (1) mit einem Ventilsitz (10) und einem in Richtung auf den Ventilsitz (10) über eine vorgespannte Ventilfeder (30) mit einer Schließkraft beaufschlagten Ventilkörper (20) und
einer strömungstechnischen Verbindung (40) zwischen der hydraulischen Dämpfereinrichtung und dem Ventilsitz (10),
wobei
das Ventil (1) eine Verstelleinrichtung (50) zum Verstellen der Vorspannung der Ventilfeder (30) aufweist, **dadurch gekennzeichnet, dass** die Verstelleinrichtung (50) einen motorischen Antrieb (80) und ein mit dem motorischen Antrieb (80) gekoppeltes Steuergerät (85) zum Steuern des motorischen Antriebes (80) sowie zumindest einen mit dem Steuergerät (85) gekoppelten Sensor (95) aufweist und das Steuergerät (85) eingerichtet ist, die Federvorspannung in Abhängigkeit zumindest eines Sensorwertes zu verstellen.

2. Orthopädietechnische Vorrichtung nach Anspruch 1, **dadurch gekennzeichnet, dass** die Verstelleinrichtung (50) einen Gewindetrieb aufweist.

3. Orthopädietechnische Vorrichtung nach Anspruch 2, **dadurch gekennzeichnet, dass** die Verstelleinrichtung (50) einen mit der Ventilfeder (30) gekoppelten Ventilfederhalter (60) aufweist, der in einem Gehäuse (70) gelagert und an dem ein erstes Gewinde (65) ausgebildet ist, das mit einem zweiten Gewinde (75), das in dem Gehäuse (70) angeordnet ist, in Eingriff steht.

4. Orthopädietechnische Vorrichtung nach einem der voranstehenden Ansprüche, **dadurch gekennzeichnet, dass** die Vorspannkraft auf den Ventilkörper (20) stufenlos einstellbar ist.

5. Orthopädietechnische Vorrichtung nach einem der voranstehenden Ansprüche, **dadurch gekennzeichnet, dass** die Verstelleinrichtung (50) Einstellmarkierungen (55) aufweist.

6. Orthopädietechnische Vorrichtung nach einem der voranstehenden Ansprüche, **dadurch gekennzeichnet, dass** die Verstelleinrichtung (50) ein Formschlusselement (56) aufweist, über das die Verstelleinrichtung (50) betätigbar ist.

7. Orthopädietechnische Vorrichtung nach Anspruch 1, **dadurch gekennzeichnet, dass** die orthopädietechnische Vorrichtung ein mit dem Steuergerät (85) gekoppeltes Bedienelement (90) und/oder eine Kommunikationsschnittstelle (91) zum Ansteuern des motorischen Antriebes (80) aufweist.

8. Orthopädietechnischen Vorrichtung nach Anspruch 1, **dadurch gekennzeichnet, dass** das Steuergerät (85) dazu eingerichtet ist, das Körpergewicht und/oder einen Wert eines Parameters einer Bewegung des Nutzers mittels zumindest eines Sensors (95) zu ermitteln, aus dem ermittelten Wert oder den ermittelten Werten zumindest einen Steuerparameter für den Antrieb (80) zu errechnen und automatisch den Antrieb (80) zum Verstellen der Vorspannung der Ventilfeder (30) zu aktivieren.

## Claims

1. An orthopaedic device with
a hydraulic damping device,
a valve (1) with a valve seat (10) and a valve body (20) that is subjected to a closing force in the direction of the valve seat (10) via a pre-loaded valve spring (30), and
a fluidic connection (40) between the hydraulic damping device and the valve seat (10),
wherein
the valve (1) has an adjustment device (50) for adjusting the preload of the valve spring (30), **characterised in that** the adjustment device (50) has a motor drive (80) and a control unit (85) coupled to the motor drive (80) for controlling the motor drive (80) and at least one sensor (95) coupled to the control unit (85), and the control unit (85) is set up to adjust the spring preload as a function of at least one sensor value.

2. Orthopaedic device according to claim 1, **characterised in that** the adjustment device (50) comprises a screw drive.

3. Orthopaedic device according to claim 2, **characterised in that** the adjustment device (50) has a valve spring holder (60) which is coupled to the valve spring (30) and is mounted in a housing (70) and on which a first thread (65) is formed which engages with a second thread (75) which is arranged in the housing (70).

4. Orthopaedic device according to one of the preceding claims, **characterised in that** the preload force on the valve body (20) is continuously adjustable.

5. Orthopaedic device according to one of the preceding claims, **characterised in that** the adjustment device (50) has adjustment markings (55).

6. Orthopaedic device according to one of the preceding claims, **characterised in that** the adjustment device (50) has a positive-locking element (56) via which the adjustment device (50) can be actuated.

7. Orthopaedic device according to claim 1, **characterised in that** the orthopaedic device has an operating element (90) coupled to the control device (85) and/or a communication interface (91) for controlling the motor drive (80).

8. Orthopaedic device according to claim 1, **characterised in that** the control device (85) is set up to determine the body weight and/or a value of a parameter of a movement of the user by means of at least one sensor (95), to calculate at least one control parameter for the drive (80) from the determined value or the determined values and to automatically activate the drive (80) for adjusting the preload of the valve spring (30).

## Revendications

1. Dispositif orthopédique, comprenant
un organe d'amortissement hydraulique,
une valve (1) ayant un siège de valve (10) et un corps de valve (20) soumis à une force de fermeture en direction du siège de valve (10) par l'intermédiaire d'un ressort de valve précontraint (30), et
une communication fluidique (40) entre l'organe d'amortissement hydraulique et le siège de valve (10),
la valve (1) présentant un organe de réglage (50) pour régler la précontrainte du ressort de valve (30),
**caractérisé en ce que**
l'organe de réglage (50) comprend un entraînement motorisé (80) et un appareil de commande (85) couplé à l'entraînement motorisé (80) pour commander l'entraînement motorisé (80), ainsi qu'au moins un capteur (95) couplé à l'appareil de commande (85), et
l'appareil de commande (85) est conçu pour régler la précontrainte du ressort en fonction d'au moins une valeur du capteur.

2. Dispositif orthopédique selon la revendication 1,
**caractérisé en ce que** l'organe de réglage (50) comprend un mécanisme à vis.

3. Dispositif orthopédique selon la revendication 2,
**caractérisé en ce que** l'organe de réglage (50) comprend un support de ressort de valve (60) qui est couplé au ressort de valve (30), qui est monté dans un boîtier (70) et sur lequel est formé un premier filet (65) qui est en prise avec un deuxième filet (75) disposé dans le boîtier (70).

4. Dispositif orthopédique selon l'une des revendications précédentes,
**caractérisé en ce que** la force de précontrainte sur le corps de valve (20) est réglable en continu.

5. Dispositif orthopédique selon l'une des revendications précédentes,
**caractérisé en ce que** l'organe de réglage (50) présente des repères de réglage (55).

6. Dispositif orthopédique selon l'une des revendications précédentes,
**caractérisé en ce que** l'organe de réglage (50) comprend un élément de coopération de forme (56) permettant d'actionner l'organe de réglage (50).

7. Dispositif orthopédique selon la revendication 1,
**caractérisé en ce que** le dispositif orthopédique présente un élément de manipulation (90) couplé à l'appareil de commande (85) et/ou une interface de communication (91) pour piloter l'entraînement motorisé (80).

8. Dispositif orthopédique selon la revendication 1,
**caractérisé en ce que** l'appareil de commande (85) est conçu pour déterminer le poids du corps et/ou une valeur d'un paramètre d'un mouvement de l'utilisateur au moyen d'au moins un capteur (95), pour calculer au moins un paramètre de commande de l'entraînement (80) à partir de la valeur déterminée ou des valeurs déterminées, et pour activer automatiquement l'entraînement (80) afin de régler la précontrainte du ressort de valve (30).
